# EUROPEAN PATENT SPECIFICATION

(11) **EP 1 907 844 B1**
(45) Date of publication and mention of the grant of the patent: **03.04.2013**
(21) Application number: 06747312.4
(22) Date of filing: 09.06.2006
(51) Int. Cl.: G01N 33/53, C12N 15/09, G01N 37/00

(54) **METHOD OF MANUFACTURING PROBE-IMMOBILIZED CARRIER**
VERFAHREN ZUR HERSTELLUNG EINES SONDENIMMOBILISIERTEN TRÄGERS
PROCÉDÉ PERMETTANT DE FABRIQUER UN PORTEUR IMMOBILISÉ PAR UNE SONDE

(30) Priority: 10.06.2005 JP 2005170777
(43) Date of publication of application: 09.04.2008
(73) Proprietor: Canon Kabushiki Kaisha, Ohta-ku Tokyo (JP)
(72) Inventor: KAWAMURA, Masashi, c/o CANON KABUSHIKI KAISHA, Tokyo 1468501 (JP); OKABE, Tetsuo, c/o CANON KABUSHIKI KAISHA, Tokyo 1468501 (JP)
(74) Representative: Weser, Wolfgang
(86) International application number: PCT/JP2006/312092
(87) International publication number: WO 2006/132446

(56) References cited:
- EP-A- 0 913 690
- JP-A- 11 083 857
- JP-A- 2003 028 867
- JP-A- 2004 101 516
- JP-A- 2004 271 337
- JP-A- 2005 140 576

### SEQUENCE LISTING

<110> CANON KABUSHIKI KAISHA
<120> method of manufacturing probe-immobilized carrier
<130> 10026029W001
<150> JP 2005-170777
   <151> 2005-06-10
<160> 2
<170> Patent In version 3.3
<210> 1
   <211> 18
   <212> DNA
   <213> Artificial
<220>
   <223> Probe
<400> 1
   actggccgtc gttttaca 18
<210> 2
   <211> 18
   <212> DNA
   <213> Artificial
<220>
   <223> Probe
<400> 2
   actggccgtc gttttaca 18

## Description

### TECHNICAL FIELD

The present invention relates to a method of manufacturing a probe-immobilized carrier having a probe immobilized on a substrate and capable of detecting a target substance.

### BACKGROUND ART

As one of technologies for quickly and precisely determining a base sequence of a nucleic acid, detecting a nucleic acid having a specific target base sequence in a specimen, and identifying various bacterial species, there is proposed a use of a probe-immobilized carrier (probe array) having a number of probes arranged on a solid-phase support. The term "probe" as used herein means a substance specifically bound to a target nucleic acid by a hybridization reaction, which may be referred to as a probe nucleic acid when the substance is a nucleic acid. Various methods have been known as those to be used for immobilizing probes on a solid-phase support. To be specific, for example, there are exemplified a method of carrying out the immobilization of probes by a sequential synthesis of probes on a solid-phase support (i.e., an on-chip method) and a method of immobilizing previously-prepared probes by providing them on a substrate using pins, stamp, or the like. U.S. Patent No. 5143854 discloses a method by which protective groups are removed from a selected area of the substrate by an activator and a monomer having a removable protective group is repeatedly bound to the area to synthesize polymers having various sequences on the substrate. Further, for example, Japanese Patent Application Laid-open No. H08-023975 discloses a method by which a material for immobilizing a probe composed of a substrate and a material for immobilization made of a polymeric compound having a carbodiimide group carried on the substrate, is brought into contact with a biologically active substance having reactivity with a carbodiimide group to thereby immobilize the probe on the substrate. Still further, for example, Japanese Patent Application Laid-open No. 2001-178442 discloses a method by which a DNA fragment having a thiol group at a terminal thereof is brought into contact in a liquid phase with a solid-phase carrier in which a linear molecule having a reactive group capable of reacting with and covalently binding to the thiol group is immobilized on the surface on one terminal'. With this, the DNA fragment can be immobilized on the surface of the solid-phase carrier as the DNA and the linear molecule can be covalently bound to each other. Yet further, for example, Japanese Patent Application Laid-open No. 2000-295990 describes a method by which an aqueous solution prepared by dissolving or dispersing both a DNA fragment and a hydrophilic polymer into an aqueous medium is spotted on the surface of a solid-phase carrier to stabilize the binding of the DNA fragment with the surface of the-solid-phase carrier.

The probe array prepared as described above is generally desired to be of high sensitivity. This is because a question may arise for the reliability of the detection results due to a decrease in S/N ratio etc when the concentration of a target substance to be detected by the probe array is low. Therefore, attempts have been conducted on a method of improving the sensitivity of the probe array by raising the concentration of the probe, to thereby increase the amount of the probe to be immobilized on the substrate.

However, as for the probe array thus prepared as described above, the amount of probe bound may become saturated with respect to the amount of reactive group, which can be bind to the probe on the substrate. As a result, in a liquid droplet containing the probe spotted on the substrate, an unreacted probe may remain as it is. Further, when the liquid droplet in such a condition is removed by liquid phase treatment with water, a detergent, or the like, the unreacted probe may flow to an area other than the area on which the unreacted probes have been spotted ("spot area"). As a result, the probe can be immobilized on a reactive group that resides on the area ("background area") other than the spot area of the substrate, thereby leading to the cause of nonspecific adsorption. The term "nonspecific adsorption" represents the adsorption or bond of the target nucleic acids at positions, which are not relevant to the detection and irrespective of manners of the adsorption and bond. In addition, when a spotted liquid droplet is removed in the liquid phase while the unreacted probe remains therein, the probe flown out may contaminate the adjacent spot area. As a result, the spot area in which only one species of the probe should be immobilized may be contaminated with another species of the probe.

In addition to those facts, the substrate itself, to which the probe can bind, has a factor leading to nonspecific adsorption of the probe thereon, so a problem may arise. In other words, when a target substance is nonspecifically adsorbed on the entire background area of the probe array, the boundary between the spot and the background area around the spot cannot be found anymore, resulting in impossibility to decide whether it is a detection signal or not. This problem occurs, for example, when a reactive group showing a positive charge in an aqueous solution, such as an amino group, exists on the substrate, is electrostatically adsorbed on the negative charge of target nucleic acid.

Conventionally, for solving those problems, a method of preventing a target substance from nonspecific adsorption to the background area of a probe array has been attempted.

For preventing the target substance from the nonspecific adsorption to the background area, the known treatment of probe array is to carry out a blocking treatment on the probe array using skim milk or the like. It is also known that a blocking treatment can be carried out such that a probe is dipped into an aqueous polymer solution after being immobilized on a substrate. For instance, as described in Japanese Patent No. 2794728, there is a method of carrying out a blocking treatment by immobilizing a probe onto a nitrocellulose film and then dipping the film into a solution containing PVA and/or PVP.

However, even if any of those blocking agent is used, the blocking agent is bound to the solid-phage support by adsorption but not by chemical bonding. Thus, the blocking effect is not necessarily enough so that the results have not always been reproducible. Further, this method cannot avoid the nonspecific adsorption of unreacted probe in the step of preparing a probe array.

In contrast, as an example of the blocking with a chemical bond, a method of blocking a poly-L-lysine-coated slide glass with succinic anhydride has been reported (see, e.g., P. O. Brown et al., Genome Res., 6: 639-645, 1996). This is a method of attempting elimination of a charge from an amino group on the slide glass by coupling the amino group with succinic anhydride. Even in this method, however, when an unreacted probe remains in a liquid droplet spotted on the solid-phase support in the step of preparing a probe array, the unreacted probe may flow to the background area during the blocking reaction so that the blocking agent can be competitively reacted with the probe flown out, thereby leading to nonspecific adsorption.

EP 0 913 690 A discloses the use of polyalkylene oxide modified reagents in a method for the detection of an analyte or in suitable reagent kits for such methods.

### DISCLOSURE OF THE INVENTION

The present invention provides a method of manufacturing a probe-immobilized carrier that detects a target substance (target), where a spot is prevented from being contaminated with another spot and a probe is prevented from nonspecifically adsorbing a background area in the manufacture of the probe-immobilized carrier and nonspecific adsorption cannot be occurred even after the formation of an array.

In other words, the present invention provides the method of manufacturing a probe-immobilized carrier according to claim 1. The other claims relate to further developments.

The conventional blocking method may lead to nonspecific adsorption as spotted DNA flows out during the blocking reaction. In contrast, the present invention enables the inactivation of a background area without changing the form of a liquid droplet after supplying the liquid droplet..

Other features and advantages of the present invention will be apparent from the following - description taken in conjunction with the accompanying drawings, in which like reference characters designate the same or similar parts throughout the figures thereof.

### BRIEF DESCRIPTION OF THE DRAWINGS

Fig. 1 illustrates exemplified blocking compounds when a thiol-labeled probe is used..
Fig. 2 illustrates exemplified blocking compounds when an amino-labeled probe is used.
Fig. 3 is a diagram showing blocking effects of 1-propanethiol with respect to a concentration of the probe.
Fig. 4 is a diagram showing the blocking effects of ethanol amine with respect to the concentration of the probe.

### BEST MODE FOR CARRYING OUT THE INVENTION

Preferred embodiments of the present invention will now be described in detail in accordance with the accompanying drawings.

The present invention relates to a method of manufacturing a probe-immobilized carrier that uses a substrate containing a reactive group for immobilizing a probe thereon, including the steps of:
(i) supplying a liquid droplet containing a probe on the substrate;
(ii) inactivating a reactive group existing in an area other than a supplying area of the substrate, and
(iii) removing an unreacted probe existing in the supplied liquid droplet.

The above supply is preferably carried out by spotting the liquid droplet. In this case, the characteristic feature of the present invention is to carry out the inactivation by means that retains the form of the supplied liquid droplet. To be specific, the characteristic feature of the present invention is to inactivate a reactive group that can bind to a probe on an external area (background area) of the spotted portion (spot area) while the form of the liquid droplet spotted on the substrate is retained as it is.

In this case, the above step (ii) may include the process of carrying out the inactivation with a blocking compound. As means for carrying out blocking with a blocking compound without changing the form of the spotted liquid droplet, a gas-phase treatment, a spray treatment, or the like can be exemplified. The gas-phase treatment is particularly effective when a blocking compound which vaporizes at normal temperature or with heat from a heater or the like (e.g., a low-molecular-weight compound) is used, and the reaction group can be thus inactivated by hermetically closing both a probe-immobilized carrier which has completed the spotting and a blocking compound in a closed chamber or a closed chamber having a heather and letting them stand for a certain period of time. The spray treatment is particularly effective when a blocking compound that cannot be vaporized at normal temperature (e.g., a high-molecular-weight compound, or a polar compound having a strong intermolecular attraction) is used. The spray treatment can be attained by dissolving a blocking compound in a suitable solvent to prepare a blocking compound solution, making the blocking compound solution into mist with a spraying device such as a spray, and spraying the blocking compound solution on the probe-immobilized carrier that has completed the spotting.

Alternatively, the inactivation with the vaporized blocking compound may be carried out in a vacuum evaporator or in a simplified vacuum desiccator made of polycarbonate, and preferably carried out in a device capable of forming a vacuum space. In the case of using a blocking compound in solid form or in liquid form having a high boiling point, the vacuum space is employed to lower the boiling point, thereby allowing the blocking compound to be evaporated even at comparatively low temperatures. Therefore, this method can be effective for a heat-sensitive blocking compound.

Further, the spray treatment is preferably carried out in the process of, for example, constructing a manufacturing line in which a spray nozzle is installed and spraying a blocking compound solution from the spray nozzle so as to coat the solid-phase support on which a liquid droplet has been spotted. Preferably, at this time, the blocking compound solution ejected from the spray nozzle has a mist size of approximately 10 µm to 20 µm, ideally 1µm to 5 µm in mist form. If the mist size thereof is higher than the range, the trace of mist may be recognized and may be of visually objectionable. Besides, since the liquid droplet containing the spotted probe has a diameter of approximately 50 µm to 500 µm, the spotted liquid droplet can be physically deformed when the mist diameter is almost the same as that of the liquid droplet containing the spot probes. Therefore, it is not a favorable mist size from the view point of the intention of the present invention.

Alternatively, there is means for carrying out the blocking while holding the shape of a liquid droplet intact without using a blocking compound. For instance, after the formation of a liquid droplet on a substrate, a mask having a profile along the shape of the liquid droplet may be hung above the substrate with a spacing of several hundreds micrometers, followed by inactivating a reactive group on the background area by irradiation of high-energy electromagnetic rays or particle rays, such as X-rays, α-rays, β-rays, γ-rays, high-energy neutron rays, electron rays, vacuum ultraviolet rays, or ultraviolet rays.

Further, the step of removing an unreacted probe existing in the spotted liquid droplet in accordance with the present invention is to remove the liquid droplet by liquid-phase treatment with water, a detergent, or the like. Drying after the liquid-phase treatment may be carried out such that moisture is removed from the substrate with compressed air or by spinning the substrate, followed by natural drying or by drying with heat or the like.

Further, the probes to be used in the present invention include, but are not particularly limited thereto as far as it is a substance capable of specifically binding to a standard substance, biological macromolecules such as proteins (including complex proteins), nucleic acids, sugar chains (including complex sugars), and lipids (including complex lipids). Specific examples thereof include enzymes, hormones, pheromones, antibodies, antigens, haptens, peptides, synthetic peptides, DNAs, synthetic DNAs, RNAs, synthetic RNAs, PNAs, synthetic PNAs, gangliosides, and lectins. In particular, oligonucleotides, polynucleotides, or peptide nucleic acids are preferable. Further, nucleotide derivatives or analogs thereof are also included.

In addition, when a thiol group is introduced into a probe, for example when an automatically synthesized DNA is used as a probe, Thiol-Modifier (manufactured by Glen Research Corp.) can be used for the synthesis with an automated DNA synthesizer. However, it is not particularly limited thereto as far as the thiol group can be introduced efficiently.

On the other hand, when an amino group is introduced into a probe, for example when an automatically synthesized DNA is used as a probe, Amino-Modifier (manufactured by Glen Research Corp.) can be used for the synthesis with an automated DNA synthesizer, but not particularly limited thereto as far as the amino group can be introduced efficiently.

Further, means for spotting on a solid-phase support for immobilization of a probe may be one for spotting a solution, in which a probe is dissolved or dispersed in an aqueous solution, by any of an inkjet method, a pin method, a pin-and-ring method, and the like.

Among the methods mentioned above, the inkjet method is a suitable spotting method because of its ability to carry out high-density, precise spotting. The inkjet method is a method in which a probe-containing solution is placed in an extra-fine nozzle, pressure or heat is instantaneously applied on a portion near the nozzle's tip to correctly eject an extremely low volume of the probe-containing solution from the nozzle's tip, thereby allowing it to fly through the spacing and attach to the surface of the substrates. For spotting by the inkjet method, components contained in the probe solution are not particularly limited as far as it does not substantially affect on the probe when they are ejected as those of a probe solvent and comply with the requirements for the solvent's components which can be normally ejected on a substrate using an inkjet head. For example, when the inkjet head is a bubble-jet head having a mechanism for discharging a solvent with the application of thermal energy, a preferable component to be contained in the probe solvent is a liquid containing glycerin, thiodiglycol, isopropyl - alcohol, and acetylene alcohol. Further, specifically, a liquid containing 5 to 10 wt% of glycerin, 5 to 10 wt% of thiodiglycol, and.0.5 to 1 wt% of acetylene alcohol is suitably used as a probe medium. In addition, when the inkjet head is a piezo-jet head that ejects a solution using a piezoelectric element, a preferable component to be contained in a probe solvent is a liquid containing ethylene glycol and isopropyl alcohol. More specifically, a liquid containing 5 to 10 wt% of ethylene glycol and 0.5 to 2 wt% of isopropyl alcohol is suitably used as a probe solvent.

When the probe solution obtained as described above is ejected from the inkjet head and attached on a substrate, the form of a spot is circular and no expansion of the area on which the probe solution has been ejected is caused. In addition, the connection with an adjacent spot can be effectively prevented even when the probe solution is spotted in high density. In this case, the characteristic features of the probe solution of the present invention are not limited to those described above.

Further, the'pin method described above is a method by which a probe is spotted on a substrate using a contact pin, so the probe can be immobilized with a simple equipment. Thus, it is useful to assess the feasibility of the probe.

Further, the substrates of the present invention can include inorganic materials and polymeric materials, but not particularly limited thereto, as far as they have no trouble in detection of a detection substance (target substance) using a probe-immobilized substrate prepared by immobilizing a probe on the substrate. Preferably, any of those substrates may have a reactive group, such as an amino group, a maleimide group, an acrylamide group, an N-hydro-succinimidyl ester group, a formyl group, a carboxyl group, or an epoxy group, being introduced on the surface thereof or may be one selected from materials originally containing these reactive groups, but not limited to those reactive group.

When an inorganic material is used for a substrate in particular, a substrate whose surface is treated by a silane coupling agent having an amino group, for example, is preferable in order to introduce a basic group therein. In this case, materials capable of being efficiently treated by a silane coupling agent, such as quartz, glass, silica, alumina, talc, clay, aluminum, aluminum hydroxide, iron, mica, and the like are particularly preferable, and oxides such as titanium oxide, zinc oxide, and iron oxide can also be used. An alkali-free glass containing no alkali components and the like or a crystal substrate material is particularly preferable in view of detection of target substances and its, generality as a material. Examples of the silane coupling agent having an amino group include N-β(aminoethyl)γ-aminopropyl trialkoxysilane, N-β(aminoethyl)γ-aminopropylmethyl dialkoxysilane., γ-aminopropyl trialkoxysilane, and γ-aminopropylmethyl dialkoxysilane. For an alkoxysilyl group, a methoxysilyl group or an ethoxysilyl group capable of being rapidly hydrolyzed is preferable.

Further, the polymeric materials include, preferably, those having basic groups such as an amino group or those into which basic groups can be readily introduced. For instance, there are given a method of utilizing polyamide having an amino group on its terminal end and a method of detaching a protective group by allowing a protected amino group to copolymerize with a substance having a vinyl group.

In this case, the silane-coupling agent of the present invention refers to any of compounds having' both an organic functional group which can.be reacted with an organic compound such as a resin and a portion which can be bound to an inorganic compound such as glass through a siloxane bond.

The shape of the substrate is not restricted to, but for example a DNA chip may be preferably in the shape of a substrate because of its versatility in detection methods, devices, and so on. Further, the substrate material is preferably one having a high degree of surface flatness. In particular, it is preferably a substrate having dimensions of approximately 1 inch x 3 inches and a thickness of approximately 0.7 to 1.5 mm.

Further, the blocking compound of the present invention is preferably one that contains, in a molecule, the same reactive group as that of the probe to be reacted with a substrate. Therefore, by having the same reactive group as that of the probe in the molecule of the blocking compound, such a blocking compound has an advantage in that it can be easily controlled because the blocking reaction of a background area can be carried out by the same mechanism as that of the binding method of immobilizing the probe on the substrate.

Further, it is preferable that the blocking compound of the present invention has a chemical basic skeleton which does not adsorb to a target substance. Further, to prevent the blocking compound from interaction with a target substance after completing the blocking reaction, the blocking compound may preferably have a single reactive group in molecule. However, the blocking compound having two or more active groups in molecule may be used as far as the active groups are kind of having no interaction with a target substance.

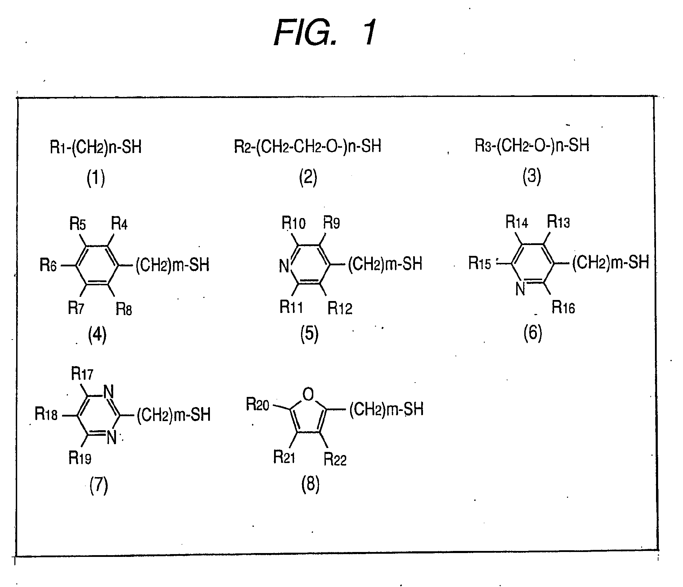

For a specific blocking compound, when a reactive group of the probe is a thiol group, a compound having the chemical basic structure as shown in Fig. 1 is desirable. In Fig. 1, n = 0 to 100, m = 0 to 25, R₁ to R₂₂ are each independently selected from the group consisting of H, OH, CH₃, NH₂, CH₂-CH₃, CH=CH₃, X, CH₂X, CHX₂, CH₂-CH₂X, CX₃, CX₂-(CX₂)ₘ-CX₃, O-(CH₂)ₘ-CH₃, (CH₂)ₘ-OH, (CH₂)ₘ-C(=O)-OH, (CH₂)ₘ-NH₂, (CH₂)ₘ-SH, and C(=O)-(CH₂)ₘ-CH₃, and each of X is selected from halogens; provided that moieties each corresponding to (CX₂)ₘ and (CH₂)ₘ may be branched with regard to each of CX₂-(CX₂)ₘ-CX₃, O-(CH₂)ₘ-CH₃, (CH₂)ₘ-OH, (CH₂)ₘ-C(=O)-OH, (CH₂)ₘ-NH₂, and (CH₂)ₘ-SH, and each of branched ends is selected from the group consisting of CX₃, CH₃, OH, C(=O)-OH, C(=O)-H, NH₂, and SH.

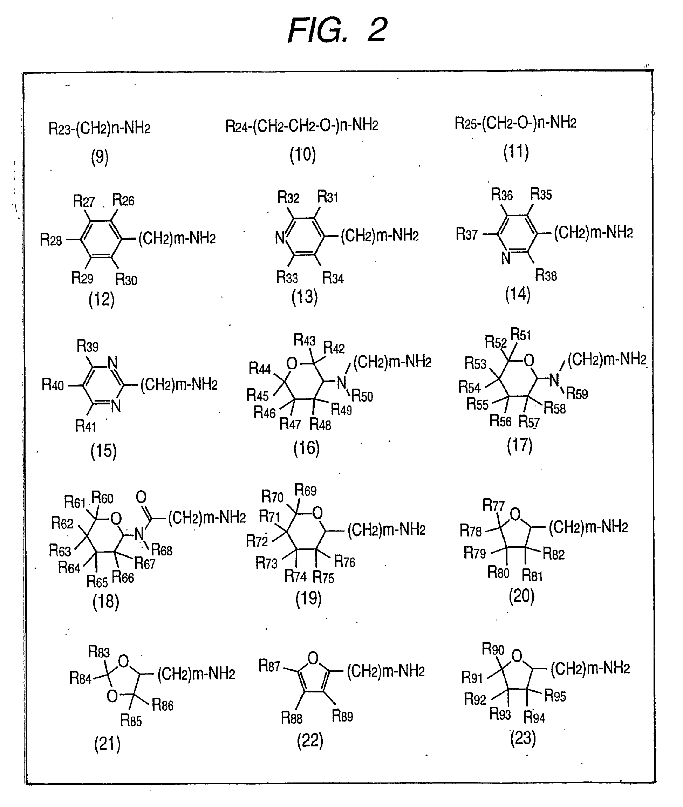

In addition, when a reactive group of the probe is an amino group, a compound having the chemical basic structure as shown in Fig. 2 is desirable. In Fig. 2, n = 0 to 100, m = 0 to 25, R₂₃ to R₉₅ are each independently selected from the group consisting of H, OH, CH₃, NH₂, CH₂-CH₃, CH=CH₃, X, CH₂X, CHX₂, CH₂-CH₂X, CX₃, CX₂-(CX₂)ₘ-CX₃, O-(CH₂)ₘ-CH₃, (CH₂)ₘ-OH, (CH₂)ₘ-C(=O)-OH, (CH₂)ₘ-NH₂, (CH₂)ₘ-SH, and C(=O)-(CH₂)ₘ-CH₃, and each of X is selected from halogens; provided that moieties each corresponding to (CX₂)ₘ and (CH₂)ₘ may be branched with regard to each of CX₂-(CX₂)ₘ-CX₃, O- (CH₂)ₘ-CH₃, (CH₂)ₘ-OH, (CH₂)ₘ-C(=O)-OH, (CH₂)ₘ-NH₂, and (CH₂)ₘ-SH, and each of branched end is selected from the group consisting of CX₃, CH₃, OH, C(=O)-OH, C(=O)-H, NH₂, and SH.

Further, the reaction of any of these blocking compounds with the surface of a substrate may be confirmed by a surface analysis method by which a fragment-detecting value from the TOF-SIMS (Time of Flight - Secondary Ion Mass Spectrometry) or the like is represented in two dimensional profile and then analyzed. To be specific, a probe-immobilized carrier, on which a blocking reaction has been completed, is rinsed with purified water and then dried with N₂ blowing or the like. Subsequently, the resulting product is subjected to a surface analysis with TOF-SIMS. By focusing attention on a sulfur (S), atom with respect to a combination of a thiol-labeled probe with a blocking compound containing a thiol group and by focusing attention on a nitrogen (N) atom with respect to a combination of an amino-labeled probe with a blocking compound containing an amino group, fragment-detecting values of the probe-immobilized carriers are represented in two-dimensional profiles and then analyzed, respectively. As a result, the detection values for S or N atom in spot and non-spot areas are found such that a difference between the detection values decreases in comparison with those of unblocked probe-immobilized carriers. Further, in this analysis method, if the reactive group of the probe is the same as that of the blocking compound, it is possible to focus attention on a fragment specific to the reactive group and analyze without being restricted on a sulfur (S) or nitrogen (N) atom.

### [Examples]

### <Example 1> Blocking when thiol-labeled DNA probe is used

### (i) Synthesis of probe and synthesis of fluorescent-labeled target substance (target)

As a probe capable of specifically binding to a target substance, a single-stranded DNA.probe was used. The single-stranded nucleic acid of SEQ ID No. 1 was synthesized using an automated DNA synthesizer. In addition, a mercapto (SH) group was introduced on the terminal of the single-stranded DNA of SEQ ID No. 1 using the Thiol-Modifier (manufactured by Glen Research Corp.) when synthesizing with the automated DNA synthesizer. Subsequently, a normal deprotection process was carried out and the DNA was then recovered and purified using high-performance liquid chromatography, followed by carrying out the experiments described below.
SEQ ID No. 1.:
   5' -HS- (CH₂)₆-O-PO₂-O-ACTGGCCGTCGTTTTACA-3'

Further, an unlabeled single-stranded DNA having a base sequence complementary to the single-stranded DNA probe of SEQ ID No. 1 described above was synthesized by the automated DNA synthesizer and Cy3 was then bound to the 5' terminal of the single-stranded DNA probe to thereby obtain a labeled single-stranded DNA (target).

### (ii) Preparation of probe-immobilized carrier

### [Washing of base plate]

As a substrate for a probe-immobilized carrier, a base plate made of synthetic quartz glass of a one-inch by three-inch square was used. The quartz glass base plate was washed as follows: Brush-washing with purified water, rinsing with purified water, ultrasonic cleaning with alkaline detergent, rinsing with purified water, ultrasonic cleaning with purified water, rinsing with purified water, and drying with N₂-blowing were carried out according to the conventional procedures, thereby preparing a quartz glass base plate having a cleaned surface.

### [Surface treatment]

An amino-silane coupling agent (trade name: KBM-603, manufactured by Shin-Etsu Chemical Co., Ltd.) was dissolved so as to be of 1 wt% and then stirred for 30 minutes to allow a methoxy group to be hydrolyzed. In the resulting aqueous solution, a slide glass was dipped for 30 minutes (heated at 8.0°C in hot bath) and then'pulled out and washed with purified water, followed by subjecting to a 1-hour baking treatment at 120°C in an oven.

Subsequently, 2.7 mg of N-maleimido-caproyloxysuccinimide (manufactured by DOJINDO LABORATORIES., hereinafter abbreviated as EMCS) was weighed and dissolved in a dimethylsulfoxide (DMSO)/ethanol (1:1) solution so as to be of a finial concentration of 0.3 mg/ml, thereby preparing an EMCS solution. The amino-group-introduced quartz glass base plate; which had been subjected to the baking treatment, was dipped in the EMCS solution for 2 hours at room temperature to introduce a maleimide group on the surface of the base plate. After the treatment with the EMCS solution, the base plate was sequentially washed with a DMSO/ethanol mixture solution and ethanol and then dried under nitrogen atmosphere.

### [Immobilization of probe]

A single-stranded DNA probe fragment (SEQ ID No. 1) synthesized in the above (i) was dissolved in an aqueous solution containing 7.5 wt% of grycerine, 7.5 wt% of urea, 7.5 wt% of thiodiglycol, and 1.0 wt% of acetylene alcohol (trade name: Acetylenol E100, manufactured by Kawaken Fine Chemicals Co., Ltd.). Five different kinds of the above aqueous solution were prepared so that the probe concentrations thereof were 8.75, 26.25, 43.75, 61.25, and 87.5 µM, respectively. In this case, it had been known that the saturated concentration of the thiol-labeled probe in reaction is approximately 50 µM with respect to the amount of a maleimide group on the base plate. An ink tank for a bubble-jet printer (trade name: BJF-850, manufactured by Canon Inc.) was filled with the probe-containing solution and then mounted on a print head. In this case, the bubble-jet printer used is modified so that it is able to carry out inkjet printing on a flat plate. In addition, the modified bubble-jet printer is able to spot an approximately 5-pl droplet of DNA solution with a pitch of approximately 120 µm by entering printing patterns into the printer in accordance with the predetermined file-creating method. Subsequently, the modified bubble-jet printer was used for spotting of the probe DNA solution on the surface of the glass base plate. Subsequently, the base plate was left standing in a thermohygrostat chamber to allow the probe to react with the base plate so as to be immobilized thereon, thereby resulting in a probe-immobilized carrier.

### (iii) Blocking of probe-immobilized carrier

The blocking agent was investigated using 1-propanethiol, the compound (1) represented in Fig. 1 with R₁ = CH₃ and n = 2.

For the blocking, a petri dish containing 10 ml of 1-propanethiol was placed in a closed chamber. Subsequently, a probe-immobilized carrier on which the spotting solution prepared in the above (ii) was being retained thereon was installed in a cassette and the cassette was then placed and sealed in the above closed chamber. After leaving it standing for 1 hour at normal temperature under such condition, a blocking reaction was carried out around the spot by a gas-phase treatment. Subsequently, the probe-immobilized carrier was washed with a 1-M NaCl/50-mM phosphate buffer solution (pH 7.0) and then lightly washed with purified water, followed by drying with nitrogen blowing to thereby obtain a probe-immobilized carrier for hybridization. In addition, for comparison, another probe-immobilized carrier was prepared without subjecting to the blocking treatment. In this case, the probe-immobilized carrier being spotted with the spotting solution prepared in the above (ii) was washed with a 1-M NaCl/50-mM phosphate buffer solution (pH 7.0). It was then lightly washed with purified water, followed by drying with nitrogen blowing.

### (iv) Hybridization and fluorescence evaluation

The fluorescence-labeled target substance synthesized in the above (i), was dissolved in a 1-M NaCl/50-mM phosphate buffer (pH 7.0) so as to be of a final concentration of 5 nM. In this solution, the washed probe-immobilized carrier was dipped and then carried out a hybridization treatment for 2 hours at a temperature of 45°C. After the treatment, the probe-immobilized carrier was washed with a 1-M NaCl / 50-mM phosphate buffer (pH 7.0) to flow out a unhybridized single-stranded DNA. It was then lightly washed with purified water, followed by drying with N₂-blowing after removal of salt content.

The fluorescence strength of the spot on the probe-immobilized carrier was determined using a fluorescence scanner (trade name: GenePix 4000B, manufactured by Axon Instruments, Inc.). In this case, the same measurement conditions were employed in both the examples and the comparative examples (532 nm in measurement wavelength for fluorescence strength).

### (v) Results

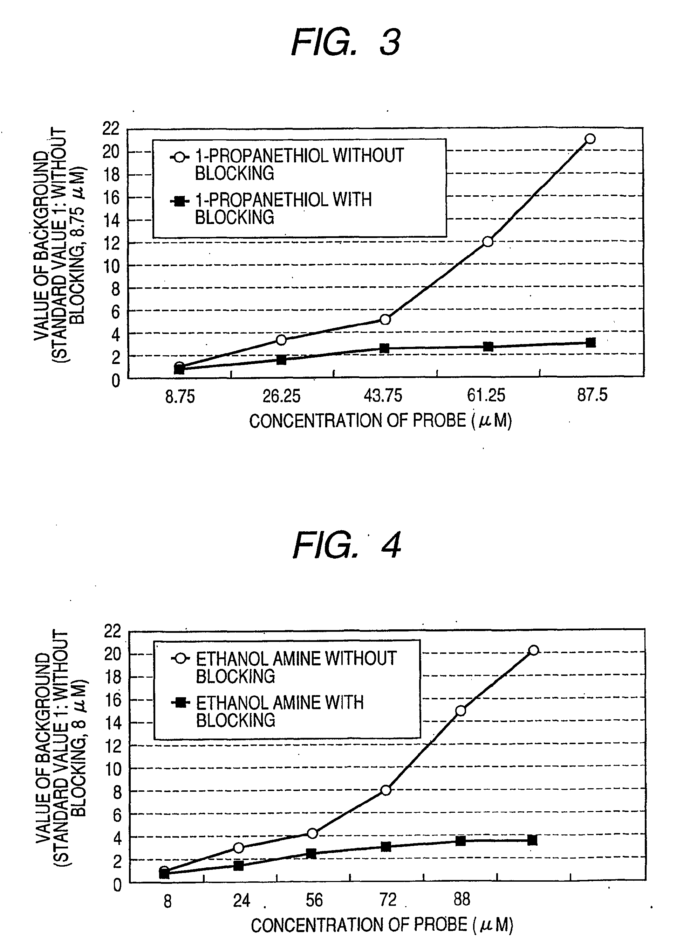

The results shown in Fig. 3 were obtained by plotting the levels of average brightness obtained from the fluorescent brightness around the spot (background) in the presence or absence of blocking with 1-propanethiol with respect to the concentrations of probe (the standard level of the background was defined under the conditions in which the probe had a lowest concentration of 8.75 µM in the absence of blocking).

Under the conditions in the absence of blocking, an abrupt increase in background can be recognized at 61.25 µM, which is not less than the saturated concentration of the probe (in the fluorescence image, it is confirmed that the spot has ran intensively). On the other hand, under the conditions in the presence of blocking, no increase in background cannot be observed even at 61.25 µM or more, which is not less than the saturated concentration of the probe. In other words, there is shown that an inactivation (blocking) with means that does not deform the shape of a spotted probe of the present invention has been attained.

### <Example 2> Blocking when amino-labeled DNA probe is used

### (i) Synthesis of probe, its complementary-strand probe, and synthesis of fluorescent-labeled target substance (target)

As a probe capable of specifically binding to a target substance, a single-stranded DNA probe was used. The single-stranded nucleic acid of SEQ ID No. 2 was synthesized using an automated DNA synthesize. In addition, an amino (NH₂) group was introduced on the terminal of the single-stranded DNA of SEQ ID No. 2 using the Amino-Modifier (manufactured by Glen Research Corp.) when synthesizing with the automated DNA synthesizer. subsequently, a normal deprotection process was carried out and the DNA was then recovered and purified using high-performance liquid chromatography, followed by carrying out the experiments described below.
SEQ ID No. 2.:
   5' -NH₂-(CH₂)₆-O-PO₂-O-ACTGGCCGTCGTTTTACA-3'

Further, an unlabeled single-stranded DNA probe having a base sequence complementary to the single-stranded DNA probe of SEQ ID No. 2 described above was synthesized by the automated DNA synthesizer and Cy3 was then bound to the 5' terminal of the.single-stranded DNA probe to thereby obtain a labeled single-stranded DNA (target).

### (ii) Preparation of probe-immobilized carrier

### [Washing of base plate]

As a substrate for a probe-immobilized carrier, a base plate made of synthetic quartz glass of a one-inch by three-inch square was used. The quartz glass base plate was washed as follows: Brush-washing with purified water, rinsing with purified water, ultrasonic cleaning with alkaline detergent, rinsing with purified water, ultrasonic cleaning with purified water, rinsing with purified water, and drying with N₂-blowing were carried out according to the conventional procedures, thereby preparing a quartz glass base plate having a cleaned surface.

### [Surface treatment]

A 50 wt% methanol aqueous solution containing 1 wt% of a silane-coupling agent (trade name: KBM403, manufactured by Shin-Etsu Chemical Co., Ltd.) including a silane compound (γ-glycidoxypropyl-trimethoxysilane) coupled with an epoxy resin was stirred for 3 hours at room temperature to allow a methoxy group in the silane compound to be hydrolyzed. Then, the solution was applied on the surface of the above base plate using a spin coater, heated at 100°C for 5 minutes, and dried to provide the surface of the base plate with an epoxy group.

### [Immobilization of probe]

In a TE solution (pH 8) containing NaCl at a concentration of 50 mM, each of amino-labeled DNA probes and unlabeled single-stranded DNA probes was dissolved to have final concentration of 200 µM, thereby preparing an amino-labeled DNA probe solution and an unlabeled single-stranded DNA probe solution. Subsequently, 100 µl of a solution containing the unlabeled single-stranded DNA probe was added to 100 µl of a solution containing the amino-labeled DNA probe and then mixed together. The resulting mixture solution was linearly cooled from 90°C to 25°C for 2 hours, thereby forming a hybrid between each of the DNA probes and each of the single-stranded nucleic acids. Subsequently, a solution containing a hybrid of the amino-labeled DNA probe of SEQ ID No. 2 described above was added to an aqueous solution containing 7.5 wt% of glycerin, 7.5 wt% of urea, 7.5 wt% of thiodiglycol, and 1.0 wt% of acetylene alcohol (trade name: Acetylenol EH, manufactured by Kawaken Fine Chemicals Co., Ltd.) to adjust the final concentration of the hybrid to each of 8, 24, 40, 56, 72, 88 and 104 µM (i.e., investigated at seven different concentrations). In this case, it had been known that the saturated concentration of the amino-labeled probe in reaction is approximately 65 µM with respect to the amount of an epoxy group on the base plate.

The probe-containing solution was spotted on the base plate by the same way as that of Example 1 and then placed for 12 hours in a thermohygrostat chamber to allow the amino group of the probe to react with the epoxy group of the base plate so as to be immobilized thereon, thereby resulting in a probe-immobilized carrier. In this case, an amino group of a base of the probe forms a hybrid with a completely complementary single DNA, so that it cannot be reacted with the epoxy group of the base plate.

### (iii) Blocking of probe-immobilized carrier

The blocking agent was investigated using ethanolamine of the compound (9) represented in Fig. 2 with R₂₃ = OH and n = 2.

For the blocking, a petri dish containing 10 ml of ethanolamine was placed in a closed chamber while retaining the petri dish in a'hot plate. A probe-immobilized carrier on which the spotting solution prepared in the above (ii) was being retained thereon was installed in a cassette and the cassette was then placed and sealed in the above closed chamber. Subsequently, the hot plate was set to 60°C to vaporize the ethanol amine from the petri dish and then left standing for 6 hours as it is to carry out a blocking reaction around the spot. Subsequently, the base plate was washed with purified water at 80°C for 10 minutes to dissociate a complementary strand hybridized with the probe bound to the base plate while washing it out, and then dried with nitrogen blowing to thereby obtain a probe-immobilized carrier for hybridization. In addition, for comparison, another probe-immobilized carrier was prepared without subjecting to the blocking treatment. In this case, the probe-immobilized carrier being spotted with the spotting solution prepared in the above (ii) was washed with purified water at 80°C for 10 minutes to dissociate a complementary strand hybridized with the probe.bound to the base plate while washing it out, and then dried with nitrogen blowing.

### (iv) Hybridization and fluorescence evaluation

The fluorescence-labeled target substance synthesized in the above (i) was dissolved in a 1-M NaCl/50-mM phosphate buffer (pH 7.0) to have a final concentration of 5 nM. In this solution, the washed probe-immobilized carrier was dipped and then subjected to a hybridization treatment for 2 hours at a temperature of 45°C. After the treatment, the probe-immobilized carrier was washed with a 1-M NaCl/50-mM phosphate buffer (pH 7.0) to wash out a unhybridized single-stranded DNA, and then lightly washed with purified water to remove salt content, followed by drying with nitrogen blowing.

The fluorescence strength of the spot on the probe-immobilized carrier was determined using a fluorescence scanner (trade name: GenePix 4000B, manufactured by Axon Instruments, Inc.). In this case, the same measurement conditions were employed in both the examples and the comparative examples (measurement wavelength for fluorescence strength: 532 nm).

### (v) Results

The results shown in Fig. 4 were obtained by plotting the levels of average brightness obtained from the fluorescent brightness around the spot (background) in the presence or absence of blocking with ethanolamine with respect to the concentrations of probe (the standard level of the background was defined under the conditions in which the probe had a lowest concentration of 8 µM in the absence of blocking).

From the results, under the conditions in the absence of blocking, an abrupt increase in background is recognized at 72 µM, which is not less than the saturated concentration of the probe (in the fluorescence image, it is confirmed that the spot has run intensively). On the other hand, under the conditions in the presence of blocking, no increase in background cannot be observed even at 72 µM or more, which is not less than the saturated concentration of the probe. Thus, there is shown that an inactivation (blocking) by means that does not deform the shape of a spotted probe of the present invention has been attained.

As is evident from the examples described above, the manufacturing method of the present invention is particularly useful when the probe is immobilized on a given period at a higher density. In other worlds, if the probe is immobilized to a higher density,' the amount of probe in a liquid droplet to be supplied should be increased (i.e., it should be concentrated much more). However, an excess amount of the probe, which does not react with the reactive group of the base plate may be generated and bound to the periphery, thereby causing a problem of more increase in background noise. This problem can be eliminated by the present invention.

Further, an embodiment of the probe-immobilized carrier manufactured in accordance with the present invention contains a first area on the surface of a carrier on which a probe nucleic acid is immobilized, and a second area having a blocking agent on the outer periphery of the first area. The first area is constructed substantially without a probe housing fixed on the first area, while the second area is constructed on the first area substantially in the absence of the probe nucleic acid fixed on the first area.

Consequently, a suitable probe-immobilized carrier having a high detection sensitivity can be obtained, where there is no nonspecific adsorption of the standard nucleic acid on the surrounding area, as well as high detection sensitivity.

The present invention is not limited to the above embodiments and various changes and modifications can be made within the scope of the present invention. Therefore to apprise the public of the scope of the present invention, the following claims are made.

This application claims priority from Japanese Patent Application No. 2005-170777 filed June 10, 2005, which can be used as further reference.

## Claims

1. A method of manufacturing a probe-immobilized carrier that uses a substrate containing a reactive group for immobilizing a probe thereon, comprising, in the indicated order, the steps of:
(i) supplying, by spotting, a liquid droplet containing a probe on the substrate;
(ii) inactivating a reactive group existing in an area other than a supplying area of the substrate, such that the form of the supplied liquid droplet is retained, by
(a) a gas-phase treatment,
(b) a spray treatment, or
(c) an irradiation of high-energy electromagnetic rays or particle rays, using a mask spaced apart from the substrate, having a profile corresponding to the shape of the liquid droplet; and
(iii) removing an unreacted probe existing in the supplied liquid droplet.

2. The method according to claim 1, alternatives (a) or (b), wherein the inactivation is carried out by a blocking compound.

3. The method according to claim 1, alternative (a), wherein the gas-phase treatment is a process by which a substrate which has completed the step (i) is enclosed in a closed chamber filled with a vaporized blocking compound.

4. The method according to claim 1, alternative (b), wherein the spray treatment is a process for spraying a blocking solution dissolving a blocking compound in a mist form onto the substrate by a spraying device.

5. The method according to claim 1, wherein the spotting is carried out by an inkjet process.

6. The method according to claim 5, wherein the inkjet process comprises a bubble-jet system.

7. The method according to claim 5, wherein the inkjet process comprises a piezo-jet system.

8. The method according to claim 1, wherein the spotting is carried out using a pin process.

9. The method according to claim 2, wherein if the reactive group that can bind to the substrate of the probe in a liquid droplet is defined as X, the blocking compound contains the reactive group X in a molecule.

10. The method according to claim 9, wherein the reactive group X is one selected from the group consisting of a thiol group, an amino group, a maleimide group, a N-hydroxysuccinimidyl ester group, a formyl group, a carboxyl group, an acrylamide group, and an epoxy group.

11. The method according to claim 2, wherein the blocking compound has a chemical structure inactive to a target substance.

12. The method according to claim 1, wherein the probe is an oligonucleotide, a polynucleotide, or a peptide nucleic acid.

13. The method according to claim 1, wherein the probe is a nucleotide derivative or its analogue.

## Patentansprüche

1. Verfahren zum Herstellen eines sondenimmobilisierten Trägers, der ein eine reaktive Gruppe enthaltendes Substrat verwendet zwecks Immobilisierens einer Sonde hierauf,
umfassend die folgenden Schritte in der angegebenen Reihenfolge:
(i) Zuführen eines Flüssigkeitströpfchens, und zwar durch Tüpfelaufbringen, zum Substrat, das eine Sonde hierauf enthält;
(ii) Inaktivieren einer reaktiven Gruppe, die vorliegt in einem Gebiet, das vom Zuführungsgebiet des Substrats verschieden ist, derart, dass die Form des zugeführten Flüssigkeitströpfchens erhalten bleibt, und zwar durch
(a) eine Gasphasenbehandlung,
(b) eine Sprühbehandlung oder
(c) Bestrahlung mit energiereichen elektromagnetischen Strahlen oder Teilchenstrahlen unter Verwendung einer vom Substrat beabstandeten Maske mit einem der Form des Flüssigkeitströpfchens entsprechendem Profil; und
(iii) Entfernen der unreagierten Sonde, die im zugeführten Flüssigkeitströpfchen vorliegt.

2. Verfahren nach Anspruch 1, Alternativen (a) oder (b), wobei das Inaktivieren durch eine blockierende Verbindung erfolgt.

3. Verfahren nach Anspruch 1, Alternative (a), wobei
die Gasphasenbehandlung vorliegt als ein Verfahren, durch das das Substrat, bei welchem der Schritt (i) abgeschlossen ist, in einer geschlossenen Kammer eingeschlossen wird, die mit einer verdampften blockierenden Verbindung gefüllt ist.

4. Verfahren nach Anspruch 1, Alternative (b), wobei
die Sprühbehandlung vorliegt als ein Verfahren zum Sprühen einer blockierenden Lösung, die eine blockierende Verbindung löst, und zwar in Sprühnebelform mittels einer Sprühvorrichtung auf das Substrat.

5. Verfahren nach Anspruch 1, wobei
das Tüpfelaufbringen durch ein Tintenstrahlverfahren erfolgt.

6. Verfahren nach Anspruch 5, wobei
das Tintenstrahlverfahren ein Blasen-Strahlsystem umfasst.

7. Verfahren nach Anspruch 5, wobei
das Tintenstrahlverfahren ein Piezo-Strahlsystem umfasst.

8. Verfahren nach Anspruch 1, wobei
das Tüpfelaufbringen unter Verwendung eines Nadel-Verfahrens erfolgt.

9. Verfahren nach Anspruch 2, wobei
die blockierende Verbindung die reaktive Gruppe X in einem Molekül enthält, wenn die reaktive Gruppe, die sich mit dem Substrat der Sonde in einem Flüssigkeitströpfchen binden kann, als X angegeben wird.

10. Verfahren nach Anspruch 9, wobei
die reaktive Gruppe X ausgewählt ist aus der Gruppe, bestehend aus einer Thiolgruppe, einer Aminogruppe, einer Maleimidgruppe, einer N-Hydroxysuccinimidylestergruppe, einer Formylgruppe, einer Carboxylgruppe, einer Acrylamidgruppe und einer Epoxygruppe.

11. Verfahren nach Anspruch 2, wobei
die blockierende Verbindung eine chemische Struktur hat, welche gegenüber einer Ziel-Substanz inaktiv ist.

12. Verfahren nach Anspruch 1, wobei
die Sonde ein Oligonucleotid, ein Polynucleotid oder eine Peptid-Nucleinsäure ist.

13. Verfahren nach Anspruch 1, wobei
die Sonde ein Nucleotid-Derivat oder dessen Analog ist.

## Revendications

1. Procédé pour la production d'un support avec une sonde immobilisée, qui utilise un substrat contenant un groupe réactif pour immobiliser une sonde sur celui-ci, comprenant, dans l'ordre indiqué, les étapes consistant à :
(i) fournir, par dépôt en taches, une gouttelette de liquide contenant une sonde sur le substrat ;
(ii) inactiver un groupe réactif existant dans la zone autre qu'une zone d'alimentation du substrat, de telle sorte que la forme de la gouttelette fournie soit maintenue, par
(a) un traitement en phase gazeuse,
(b) un traitement par atomisation, ou
(c) une irradiation avec des rayonnements électromagnétiques ou rayonnements de particules à forte énergie, en utilisant un masque espacé du substrat, ayant un profil correspondant à la forme de la gouttelette de liquide ; et
(iii) éliminer une sonde n'ayant pas réagi existant dans la gouttelette de liquide fournie.

2. Procédé suivant la revendication 1, alternative (a) ou (b), dans lequel l'inactivation est effectuée par un composé de blocage.

3. Procédé suivant la revendication 1, alternative (a), dans lequel le traitement en phase gazeuse est un procédé par lequel un substrat qui a achevé l'étape (i) est enfermé dans une chambre close hermétiquement équipée d'un composé de blocage vaporisé.

4. Procédé suivant la revendication 1, alternative (b), dans lequel le traitement par atomisation est un procédé pour atomiser une solution de blocage dans laquelle est dissous un composé de blocage sous forme d'un brouillard sur le substrat par un dispositif d'atomisation.

5. Procédé suivant la revendication 1, dans lequel le dépôt en taches est effectué par un procédé à jet d'encre.

6. Procédé suivant la revendication 5, dans lequel le procédé à jet d'encre comprend un système à jet de bulles.

7. Procédé suivant la revendication 5, dans lequel le procédé à jet d'encre comprend un système à jet piézo-électrique.

8. Procédé suivant la revendication 1, dans lequel le dépôt en taches est effectué en utilisant un procédé à broche.

9. Procédé suivant la revendication 2, dans lequel, si le groupe réactif qui peut se lier au substrat de la sonde dans une gouttelette est défini par X, le composé de blocage contient le groupe réactif X dans une molécule.

10. Procédé suivant la revendication 9, dans lequel le groupe réactif X est un groupe réactif choisi dans le groupe consistant en un groupe thiol, un groupe amino, un groupe maléimide, un groupe ester de N-hydroxysuccinimidyle, un groupe formyle, un groupe carboxyle, un groupe acrylamide et un groupe époxy.

11. Procédé suivant la revendication 2, dans lequel le composé de blocage a une structure chimique inactive vis-à-vis d'une substance cible.

12. Procédé suivant la revendication 1, dans lequel la sonde est un oligonucléotide, un polynucléotide ou un acide nucléique peptidique.

13. Procédé suivant la revendication 1, dans lequel la sonde est un dérivé de nucléotide ou son analogue.
